(19) Europäisches Patentamt
European Patent Office
Office européen des brevets

(11) Veröffentlichungsnummer : **0 082 266**
**B1**

(12) # EUROPÄISCHE PATENTSCHRIFT

(45) Veröffentlichungstag der Patentschrift : **09.01.85**

(51) Int. Cl.⁴ : **C 07 C 87/28**, C 07 C 93/14, A 61 K 31/135

(21) Anmeldenummer : **82109743.3**

(22) Anmeldetag : **22.10.82**

(54) **Substituierte 1,5-Diaminopentane, ihre Herstellung und diese enthaltende Arzneimittel.**

(30) Priorität : **02.11.81 DE 3143356**

(43) Veröffentlichungstag der Anmeldung : **29.06.83 Patentblatt 83/26**

(45) Bekanntmachung des Hinweises auf die Patenterteilung : **09.01.85 Patentblatt 85/02**

(84) Benannte Vertragsstaaten : **BE CH DE FR GB IT LI NL**

(56) Entgegenhaltungen :
**DE-A- 2 438 965**
**Chemical Abstracts Band 83, Nr. 23, 8. Dezember 1975, Columbus, Ohio, USA K.J. HOFFMANN et al. "Fibrin-stabilizing factor inhibitors. 12. 5-Dibenzylaminopentylamine and related compounds, a new type of FSF (fibrin-stabilizing factor) inhibitors" Seite 13, Spalte 2, abstract Nr. 188192e In Verbindung mit C.A. Chemical Substance Index, Band 83, Teil I-Ph, 1975**

(73) Patentinhaber : **BASF Aktiengesellschaft**
**Carl-Bosch-Strasse 38**
**D-6700 Ludwigshafen (DE)**

(72) Erfinder : **Dengel, Ferdinand, Dr.**
**Am Hirschwald 15**
**D-6901 Wilhelmsfeld (DE)**
Erfinder : **Treiber, Hans Joerg, Dr.**
**Sperberweg 1**
**D-6831 Bruehl (DE)**
Erfinder : **Seitz, Werner, Dr.**
**Bismarckstrasse 22 B**
**D-6831 Plankstadt (DE)**
Erfinder : **Mueller, Claus D., Dr.**
**Odenwaldring 84**
**D-6806 Viernheim (DE)**

**EP 0 082 266 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Die vorliegende Erfindung betrifft neue substituierte 1,5-Diaminopentane, Verfahren zu deren Herstellung sowie Arzneimittel, welche diese Substanzen enthalten.

Es ist bekannt, daß Verapamil ein supraventrikuläres Antiarrhythmikum ist, an dessen Wirkung ein Einfluß auf die atrioventrikuläre Überleitung beteiligt ist. Verapamil wirkt zudem stark calciumantagonistisch und dadurch blutdrucksenkend [vgl. : B.N. Singh, G. Elbrodt, C.T. Peter, Drugs *15*, 169-197 (1978)].

Es wurde nun gefunden, daß substituierte 1,5-Diaminopentane der Formel I

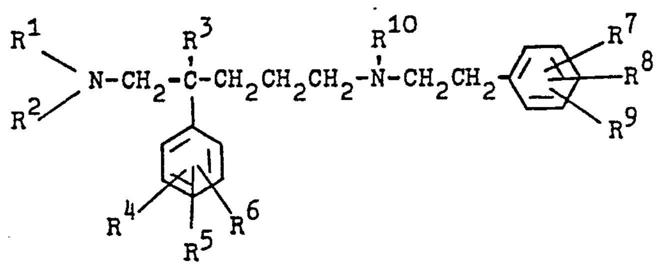

I,

worin

$R^1$ und $R^2$ Wasserstoffatome oder $C_{1-6}$-Alkylreste,

$R^3$ einen $C_{1-12}$-Alkylrest,

$R^4$-$R^9$ Wasserstoff- oder Halogenatome, $C_1$-$C_4$-Alkyl-, $C_1$-$C_4$-Alkoxy- oder Trifluormethylgruppen und

$R^{10}$ ein Wasserstoffatom oder eine Methylgruppe bedeuten,

sowie deren Salze mit physiologisch verträglichen Säuren qualitativ andere Wirkungen besitzen.

Als physiologisch verträgliche Säuren kommen Salzsäure, Schwefelsäure, Phosphorsäure, Essigsäure, Malonsäure, Bernsteinsäure, Furmarsäure, Maleinsäure, Zitronensäure, Weinsäure, Milchsäure, Amidosulfonsäure und Oxalsäure in Frage.

Die neuen Verbindungen besitzen ein asymmetrisches Kohlenstoffatom und können daher auch in Form ihrer Antipoden vorliegen.

In der Formel I bedeuten $R^1$ und $R^2$ vorzugsweise $C_{1-4}$-Alkylgruppen, insbesondere Methylgruppen. $R^3$ ist vorzugsweise eine $C_{1-4}$-Alkylgruppe. Für die Reste $R^4$-$R^9$ sind Wasserstoffatome oder Methylgruppen bevorzugt. Für $R^{10}$ ist vor allem die Methylgruppe zu nennen.

Die neuen Verbindungen lassen sich herstellen, indem man basisch substituierte Phenylacetonitrile der Formel II

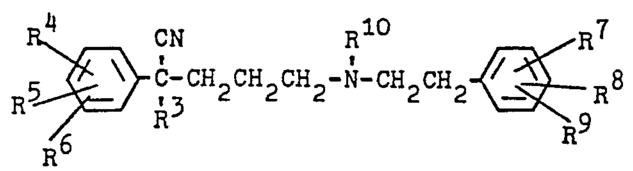

II,

worin $R^3$-$R^{10}$ die angegebene Bedeutung besitzen, reduziert und die so erhaltenen Verbindungen — falls gewünscht — mono- oder dialkyliert und gegebenenfalls anschließend in ihre Salze mit physiologisch verträglichen Säuren überführt.

Zur Reduktion der basisch substituierten Phenylacetonitrile eignen sich Reduktionsmittel, wie Diboran, oder komplexe Aluminiumhydride, z. B. Lithiumaluminiumhydrid oder Natrium-bis-(2-methoxyethoxy)-aluminium-dihydrid, wobei als Lösungsmittel besonders Tetrahydrofuran, 1,2-Dimethoxyethan, Dioxan oder Ether geeignet sind. Die Reduktion wird bei erhöhter Temperatur, vorzugsweise bei Siedetemperatur des Lösungsmittels durchgeführt.

Die Reduktion der Nitrilgruppe gelingt besonders gut durch katalytische Hydrierung mit Nickel oder Kobalt enthaltenden Katalysatoren, beispielsweise Raney-Kobalt in Gegenwart von Ammoniak. Auch Edelmetallkatalysatoren, wie Platinoxid, Palladiumschwarz, Palladium-Kohlenstoff, Ruthenium- und Rhodiumkomplexe, eignen sich. Die Hydrierung verläuft in einem Druckbereich von 50 bis 150 bar, bevorzugt bei 80 bar und in einem Temperaturbereich von 20 bis 150 °C, vorzugsweise 80 °C. Als Lösungsmittel eignen sich niedere Alkohole oder Eisessig.

Zur N-Monoalkylierung werden die primären Amine in einer hydrierenden Kondensation mit Aldehyden bzw. Ketonen umgesetzt. Mit Nickel oder Kobalt enthaltenden Katalysatoren, wie z. B. Raney-Nickel, gelingt die Reaktion besonders gut. Auch Edelmetallkatalysatoren, wie Pd/C und $PtO_2$ eignen sich. Die hydrierende Kondensation verläuft bei Normaldruck und Raumtemperatur oder wenig darüber

mit guten Ausbeuten. Die Reaktion wird vorzugsweise in niederen Alkoholen oder Eisessig durchgeführt.

Wird als Carbonylkomponente wäßrige Formaldehydlösung eingesetzt, so führt die hydrierende Kondensation zu einer N,N-Dimethylierung. Zur gleichen Verbindung gelangt man auch nach Leuckart-Wallach mittels Formaldehyd/Ameisensäure.

Die N-Monoalkylierung gelingt auch durch Umsetzung mit Säurehalogeniden bzw. -anhydriden zu den N-Acylverbindungen. Diese werden mit Borwasserstoff oder Lithiumaluminiumhydrid reduziert. Dieses Verfahren wird zur Monomethylierung bzw. -ethylierung bevorzugt. Dazu werden die primären Amine mit Formacetanhydrid bzw. Acetanhydrid in die N-Acylverbindungen überführt und diese mittels Borwasserstoff bzw. Lithiumaluminiumhydrid reduziert. Als Lösungsmittel hierfür eignen sich Ether, Tetrahydrofuran, Dioxan oder Dimethoxyethan. Die Monomethylierung läßt sich auch nach Überführung in das Carbamat mittels Chlorameisensäureester bzw. Diethoxycarbonat und anschließende Reduktion erreichen.

Die Verbindungen der Formel I lassen sich in bekannter Weise in ihre Antipoden trennen. Die Antipoden erhält man auch, wenn man von sterisch einheitlichen Ausgangsstoffen ausgeht.

Die erfindungsgemäßen Verbindungen und ihre physiologisch verträglichen Säureadditionssalze sind im Unterschied zum Verapamil weitgehend frei von calciumantagonistischen Wirkqualitäten (AV-Überleitungshemmung, Blutdrucksenkung). Statt dessen werden Effekte beobachtet, die auf einen starken Natriumantagonismus schließen lassen. Sie eignen sich daher zur Pharmakotherapie von Herzrhythmusstörungen, insbesondere ventrikulärer Genese.

Zur Bestimmung der antiarrhythmischen Wirksamkeit werden die Substanzen Ratten (Stamm : Sprague Dawley, Gewicht 200 bis 250 g) oral appliziert. 45 min später werden die Tiere mit Thiobutabarbital-Natrium (100,0 mg/kg i. p.) narkotisiert. Als arrhythmogene Substanz dient Aconitin, das 60 min nach Substanzapplikation i. v. infundiert wird (Dosierungsgeschwindigkeit : 0,005 mg/kg $\times$ min). Bei nicht behandelten Tieren (N = 52) treten nach 2,74 $\pm$ 0,07 min im EKG Arrhythmien auf, deren Eintritt durch Antiarrhythmika dosisabhängig verzögert werden kann.

Aus den linearen Beziehungen zwischen log Dosis (mg/kg) der Prüfsubstanzen und der relativen Verlängerung der Aconitin-Infusionsdauer ($\Delta$ %) wird die Dosis bestimmt, welche die Infusionsdauer um 50 % verlängert (ED 50 %).

Zur weiteren Charakterisierung der Substanzen wird aus der in den Experimenten verwendeten dezimalgeometrischen Dosenfolge (Faktor $\sqrt[3]{10}$) die antiarrhythmische Wirkung der maximal tolerierten Dosis bestimmt.

Ferner wird die Dosis ermittelt, bei welcher toxische Symptome (Veränderungen des Ausgangs-EKG, Cyanose, Krämpfe) auftreten.

Als Maß für die therapeutische Breite der neuen Verbindungen wird der Quotient aus akuter toxischer Dosis und antiarrhythmischer ED 50 % bestimmt.

Als Vergleichssubstanz diente das bekannte Antiarrhythmikum Chinidin.

Die erfindungsgemäßen Verbindungen (Tabelle 1) übertreffen an der Aconitinarrhythmie der Ratte Chinidin um das 1,3- (Beispiel 23 und 32) bis 6,8fache (Beispiel 11) an Wirksamkeit. Ein weiterer Vorteil ist die im Vergleich mit Chinidin höhere Wirkung bei Applikation der höchsten tolerierten Dosis (Tabelle 2). Chinidin verlängert die Aconitin-Infusionsdauer um maximal 129 %. Die in Tabelle 2 aufgeführten Verbindungen bewirken eine maximale Zunahme von 224 % (Beispiel 20) bis 423 % (Beispiel 37) bzw. 499 % (Beispiel 30). Darüber hinaus besitzen sie eine größere therapeutische Breite als Chinidin. Bei Chinidin treten bereits toxische Symptome auf, wenn das 11fache der wirksamen Dosis verabfolgt wird. Dagegen sind die toxischen Dosen der erfindungsgemäßen Substanzen 13- (Beispiel 37) bis 42mal (Beispiel 30) größer als die antiarrhythmisch wirksamen Dosen.

(Siehe Tabelle 1 Seite 4 f.)

Tabelle 1

Antiarrhythmische Wirkung bei der Ratte ; Applikation : per os

| Substanz des Beispiels Nr. | Wirksame Dosis mg/kg ED 50 % 1) | R.W. 2) |
|---|---|---|
| 1 | 29,4 | 1,48 |
| 6 | 21,6 | 2,01 |
| 9 | 11,8 | 3,69 |
| 10 | 11,8 | 3,69 |
| 11 | 6,42 | 6,78 |
| 12 | 22,5 | 1,93 |
| 12 | 12,9 | 3,37 |
| 14 | 14,3 | 3,04 |
| 15 | 17,1 | 2,54 |
| 17 | 26,1 | 1,67 |
| 20 | 12,3 | 3,54 |
| 21 | 23,4 | 1,86 |
| 22 | 24,8 | 1,75 |
| 23 | 33,8 | 1,29 |
| 24 | 17,0 | 2,56 |
| 27 | 21,5 | 2,02 |
| 28 | 11,6 | 3,75 |
| 30 | 11,0 | 3,96 |
| 32 | 33,8 | 1,29 |
| 33 | 21,5 | 2,02 |
| 34 | 14,7 | 2,96 |
| 35 | 8,02 | 5,42 |
| 36 | 8,66 | 5,02 |
| 37 | 7,99 | 5,44 |
| 38 | 27,8 | 1,57 |
| 40 | 6,88 | 6,32 |
| Chinidin | 43,5 | ≡ 1,00 |

1) Dosis mg/kg, welche die Aconitin-infusionsdauer um 50 % verlängert.
2) R. W. : Relative Wirksamkeit, bezogen auf Chinidin = 1,00.

(Siehe Tabelle 2 Seite 5 f.)

Tabelle 2

Antiarrhythmische Wirkung und Toxizität bei der Ratte ; Applikation : per os

| Substanz des Beispiels Nr. | Antiarrhythmische Wirkung an der Acinitinarrhythmie: Wirksame Dosis (mg/kg) ED 50 % [1] | R.W. [2] | Maximale Wirkung [3] Dosis mg/kg) | Δ % [4] | Toxizität: Dosis (mg/kg) [5] | Q [6] |
|---|---|---|---|---|---|---|
| 10 | 11,8 | 3,69 | 100 | 243 | 215 | 18 |
| 13 | 12,9 | 3,37 | 100 | 237 | 215 | 17 |
| 14 | 14,3 | 3,04 | 100 | 287 | 215 | 15 |
| 20 | 12,3 | 3,54 | 215 | 224 | 464 | 38 |
| 30 | 11,0 | 3,96 | 215 | 499 | 464 | 42 |
| 37 | 7,99 | 5,44 | 46,4 | 423 | 100 | 13 |
| Chinidin | 43,5 | ≡ 1,00 | 215 | 129 | 464 | 11 |

[1] Dosis (mg/kg), welche die Aconitininfusionsdauer um 50 % verlängert
[2] R. W. = Relative Wirksamkeit, bezogen auf Chinidin = 1,00
[3] Wirkung der maximal tolerierten Dosis
[4] Verlängerung der Aconitininfusionsdauer Δ %
[5] Dosis, nach deren Appl. die ersten toxischen Symptome beobachtet werden
[6] Q = Toxische Dosis/ED 50 %

Die neuen Verbindungen können in üblicher Weise oral oder parenteral verabfolgt werden.

Die Dosierung hängt vom Alter, Zustand und Gewicht des Patienten sowie von der Applikationsart ab. In der Regel beträgt die tägliche Wirkstoffdosis zwischen etwa 0,2 und 20 mg/kg Körpergewicht bei oraler Gabe und zwischen etwa 0,02 und 2,0 mg/kg Körpergewicht bei parenteraler Gabe. Im Normalfall werden tägliche Dosen von 0,5 bis 2 mg/kg oral und 0,05 bis 0,25 mg/kg parenteral angewendet.

Die neuen Verbindungen können in den gebräuchlichen galenischen Applikationsformen fest oder flüssig angewendet werden, z. B. als Tabletten, Filmtabletten, Kapseln, Pulver, Granulate, Dragees, Suppositorien oder Lösungen. Diese werden in üblicher Weise hergestellt. Die Wirkstoffe können dabei mit den gebräuchlichen galenischen Hilfsmitteln wie Tablettenbindern, Füllstoffen, Konservierungsmitteln, Tablettensprengmitteln, Fließregulierungsmitteln, Weichmachern, Netzmitteln, Dispergiermitteln, Emulgatoren, Lösungsmitteln, Retardierungsmitteln und/oder Antioxidantien verarbeitet werden (vgl. H. Sucker et al., Pharmaceutische Technologie, Thieme-Verlag, Stuttgart, 1978). Die so erhaltenen Wirkstoffe enthalten den Wirkstoff normalerweise in einer Menge von 0,1 und 99 Gew.%.

## Beispiel 1 bis 8

1. 1-Amino-2-isopropyl-2-phenyl-5-(phenethyl)methylaminopentan

334,5 g (1 Mol) 2,7-Dimethyl-3-cyan-3,9-diphenyl-7-azanonan werden in 6 l Methanol, das zuvor in der Kälte mit Ammoniak gesättigt wurde, gelöst und in Gegenwart von 200 g methanolfeuchtem Raney-Kobalt in einem 10-l-Rührautoklaven bei einer Temperatur von 80 °C und einem Wasserstoffdruck von 101 bar hydriert. Nach 3 h ist die Reaktion beendet. Nach Abkühlen auf Raumtemperatur wird der Katalysator abgesaugt, mit Methanol gewaschen und das Filtrat zur Trockene eingedampft. Es verbleiben 335 g Rückstand, der in Ethanol gelöst wird. Durch Zugabe von ethanolischer Oxalsäure und nach Umkristallisieren aus Ethanol werden 450 g (86 %) 1-Amino-2-isopropyl-2-phenyl-5-(phenethyl)methylaminopentan (1) als Dihydrogenoxalat erhalten, Fp. 136-138 °C. Das Dihydrochlorid schmilzt bei 266-268 °C.

Analog erhält man :

2. 1-Amino-2-isopropyl-2-(3,4-dimethoxyphenyl)-5-[(3,4-dimethoxyphenethyl)methylamino]-pentan-dihydrochlorid, FP = 220-224 °C,
3. 1-Amino-2-isopropyl-2-(3,4,5-trimethoxyphenyl-5-[(3,4-dimethoxyphenethyl)methylamino]-pentan-dihydrochlorid, Fp = 196,5-198 °C,
4. 1-Amino-2-isopropyl-2-(3-trifluormethylphenyl)-5-[(3,4-dimethoxyphenethyl)methylamino]-pentan-dihydrochlorid, Fp = 235-240 °C,
5. 1-Amino-2-isopropyl-2-(3-trifluormethylphenyl)-5-(phenylethyl)methylamino-pentan-dihydrochlorid, Fp = 252 °C,
6. 1-Amino-2-isopropyl-2-phenyl-5-(phenethyl)amino-pentan-dihydrochlorid, Fp = 225 °C,
7. 1-Amino-2-isopropyl-2-(3-trifluormethylphenyl)-5-(phenethyl)amino-pentan-dihydrochlorid, Fp = 206 °C,
8. 1-Amino-2-(n-octyl)-2-phenyl-5-(phenethyl)amino, $Kp_{0,01}$ = 220-230 °C.

## Beispiel 9 bis 27

0,1 Mol primäres 1,5-Diamino-pentan der Formel I ($R^1 = R^2 = H$), erhalten gemäß Beispiel 1, und 0,1 Mol Aldehyd bzw. Keton werden in 150 ml Methanol gelöst und bei Raumtemperatur in Gegenwart von 3 g 10 %igen Pd/C bis zur Aufnahme von 1 Äquivalent Wasserstoff hydriert.

Nach Absaugen des Katalysators wird das Methanol im Vakuum entfernt und der Rückstand durch Salzbildung und anschließende Kristallisation oder durch Chromatographie gereinigt.

Folgende Verbindungen wurden so hergestellt :

9. 1-Ethylamino-2-isopropyl-2-phenyl-5-(phenethyl)-methylamino-pentan-dihydrochlorid, Fp = 229-232 °C,
10. 1-(n-Propyl)amino-2-isopropyl-2-phenyl-5-(phenethyl)-methylamino-pentan-dihydrochlorid, Fp = 223-225 °C,
11. 1-Isopropylamino-2-isopropyl-2-phenyl-5-(phenethyl)-methylamino-pentan-dihydrochlorid, Fp = 214-216 °C,
12. 1-(n-Butyl)amino-2-isopropyl-2-phenyl-5-(phenethyl)-methylamino-pentan-dihydrochlorid, Fp = 120 °C,
13. 1-(sec-Butyl)amino-2-isopropyl-2-phenyl-5-(phenethyl)-methylamino-pentan-dihydrochlorid, Fp = 164-166 °C,
14. 1-Isobutylamino-2-isopropyl-2-phenyl-5-(phenethyl)-methylamino-pentan-dihydrochlorid, Fp = 120-135 °C,
15. 1-Ethylamino-2-isopropyl-2-(3,4-dimethoxyphenyl)-5-[(3,4-dimethoxyphenethyl)methylamino]-pentan-dihydrochlorid, Fp = 207-208 °C,
16. 1-(n-Propyl)amino-2-isopropyl-2-(3,4-dimethoxyphenyl)-5-[(3,4-dimethoxyphenethyl)methylamino]-pentan-dihydrochlorid, Fp = 228-230 °C,

17. 1-Isopropylamino-2-isopropyl-2-(3,4-dimethoxyphenyl)-5-[(3,4-dimethoxyphenethyl)methylamino]-pentan-dihydrochlorid, Fp = 180-183 °C,
18. 1-(n-Butyl)amino-2-isopropyl-2-(3,4-dimethoxyphenyl)-5-[(3,4-dimethoxyphenethyl)methylamino]-pentan-dihydrochlorid, Fp = 220-223 °C,
19. 1-(sec-Butyl)amino-2-isopropyl-2-(3,4-dimethoxyphenyl)-5-[(3,4-dimethoxyphenethyl)methylamino]-pentan-dihydrochlorid, Fp = 170-180 °C,
20. 1-Ethylamino-2-isopropyl-2-(3,4,5-trimethoxyphenyl)-5-[(3,4-dimethoxyphenethyl)methylamino]-pentan-dihydrochlorid, Fp = 205-208 °C,
21. 1-(n-Propyl)amino-2-isopropyl-2-(3,4,5-trimethoxyphenyl)-5-[(3,4-dimethoxyphenethyl)methylamino]-pentan-dihydrochlorid, Fp = 200-203 °C,
22. 1-Isopropylamino-2-isopropyl-2-(3,4,5-trimethoxyphenyl)-5-[(3,4-dimethoxyphenethyl)methylamino]-pentan-dihydrochlorid, Fp = 207-210 °C,
23. 1-(n-Butylamino)-2-isopropyl-2-(3,4,5-trimethoxyphenyl)-5-[(3,4-dimethoxyphenethyl)methylamino]-pentan-dihydrochlorid, Fp = 201-204 °C,
24. 1-(sec-Butyl)amino-2-isopropyl-(3,4,5-trimethoxyphenyl)-5-[(3,4-dimethoxyphenyl)methylamino]-pentan-dihydrochlorid, Fp = 193-195 °C,
25. 1-Isopropylamino-2-isopropyl-2-(3-trifluormethylphenyl)-5-[(3,4-dimethoxyphenethyl)methylamino]-pentan-dihydrochlorid, Fp = 70 °C,
26. 1-Isobutylamino-2-isopropyl-2-(3-trifluormethylphenyl)-5-[(3,4-dimethoxyphenethyl)methylamino]-pentan-dihydrochlorid, Fp = 70 °C.
27. 1-(n-Butyl)amino-2-isopropyl-2-(4-chlorphenyl)-5-[(3,4-dimethoxyphenethyl)methylamino]-pentandihydrochlorid, Fp = 188 °C.

## Beispiel 28 bis 33

a) Zu einer Lösung von 0,033 Mol 1,5-Diamino-pentan der Formel I ($R^1$ = $R^2$ = H) in 200 ml Methylenchlorid tropft man bei 0-5 °C unter Rühren 0,035 Mol Form-acetan-hydrid. Nach 20 min wird die Methylenchloridlösung mit 5 %iger Kaliumcarbonatlösung gewaschen, die organische Phase über Kalliumcarbonat getrocknet und anschließend im Wasserstrahlvakuum eingeengt. Man erhält die entsprechende N-Formylverbindung.

b) Zu einer gerührten Suspension von 10 g Lithiumaluminiumhydrid in 200 ml THF (Tetrahydrofuran) tropft man bei 40 °C innerhalb 15 min eine Lösung von 0,03 Mol N-Formylverbindung (vgl. a) in 50 ml THF. Anschließend wird noch 6 h auf 65 °C erwärmt. Nach Erkalten wird mit einem THF-Wasser-Gemisch und einer 5 %igen Kaliumcarbonatlösung hydrolysiert. Der entstehende weiße Niederschlag wird abgesaugt und mehrmals mit THF nachgewaschen. Die vereinigten THF-Lösungen werden zur Trockene eingeengt und der Rückstand durch Salzbildung bzw. durch Chromatographie gereinigt.

Folgende Verbindungen wurden so hergestellt :

28. 1-Methylamino-2-phenyl-5-(phenethyl)methylaminopentan, farbloses Öl,
29. 1-Methylamino-2-methyl-2-phenyl-5-(phenethyl)methyl-amino-pentan, farbloses Öl,
30. 1-Methylamino-2-isopropyl-2-phenyl-5-(phenethyl)methyl-amino-pentan-dihydrochlorid, Fp = 216-219 °C,
31. 1-Methylamino-2-(n-butyl)-2-phenyl-5-(phenethyl)methyl-amino-pentan, farbloses Öl,
32. 1-Methylamino-2-isopropyl-2-(3,4-dimethoxyphenyl)-5-[(3,4-dimethoxyphenethyl)methylamino]-pentan-dihydrochlorid, Fp = 175-180 °C,
33. 1-Methylamino-2-isopropyl-2-(3,4,5-trimethoxyphenyl)-5-[(3,4-dimethoxyphenethyl)methylamino]-pentan-dihydrochlorid, Fp = 152-155 °C.

## Beispiel 34 bis 40

Zu einer Lösung von 0,023 Mol eines 1,5-Diamino-pentans der Formel I ($R^1$ = $R^2$ = H) in 100 ml Methanol gibt man 0,023 Mol Oxalsäure, 0,046 Mol Formaldehyd (35 %ige wäßrige Lösung) und 3 g 10 %ige Pd/C. Die Reaktionslösung wird im Wasserbad unter Luftausschluß auf 40 °C erwärmt und unter leichtem Überdruck bis zur Aufnahme von zwei Äquivalenten Wasserstoff hydriert. Nach 40 min wird der Katalysator abgesaugt, das Filtrat an der Wasserstrahlpumpe eingeengt und der Rückstand in 200 ml Wasser gelöst. Durch Zugabe von Ammoniak setzt man das methylierte Amin frei und extrahiert es mit Ether. Nach Trocknen und Einengen der Etherlösung verbleibt die Base als farbloses Öl, aus dem nach Lösen in Ethanol das Dihydrochlorid ausgefällt wird.

Folgende Verbindungen wurden so hergestellt :

34. 1-Dimethylamino-2-phenyl-5-(phenethyl)methylamino-pentan, farbloses Öl,
35. 1-Dimethylamino-2-methyl-2-phenyl-5-(phenethyl)-methylamino-pentan, farbloses Öl,
36. 1-Dimethylamino-2-(n-butyl)-2-phenyl-5-(phenethyl)-methylamino-pentan, farbloses Öl,
37. 1-Dimethylamino-2-isopropyl-2-phenyl-5-(phenethyl)-methylamino-pentan-dihydrochlorid, Fp = 190-193 °C,

38. 1-Dimethylamino-2-isopropyl-2-(3,4-dimethoxyphenyl)-5-[(3,4-dimethoxyphenethyl)methylamino]-pentan-dihydrochlorid, Fp = 208-210 °C,

39. 1-Dimethylamino-2-isopropyl-2-(3,4,5-trimethoxyphenyl)-5-[(3,4-dimethoxyphenethyl)methylamino]-pentan-dihydrochlorid, Fp = 130 °C,

40. 1-Dimethylamino-2-(n-dodecyl)-2-phenyl-5-(phenethyl)methylamino-pentan, farbloses Öl.

Beispiel 41

1-Diethylamino-2-isopropyl-2-phenyl-5-(phenethyl)-methylamino-pentan

Zu einer Lösung von 36,7 g (0,1 Mol) 1-Ethylamino-2-isopropyl-2-phenyl-5-(phenethyl)methylamino-pentan (Beispiel 9) und 10,1 g (0,1 Mol) Triethylamin in 150 ml trockenem Ether tropft man unter Rühren bei 0 °C eine Lösung von 7,9 g (0,1 Mol) Acetylchlorid. Das Reaktionsgemisch wird 3 h bei Raumtemperatur gerührt, das ausgeschiedene Triethylamin-hydrochlorid abgesaugt, die Etherphase mehrmals mit Wasser gewaschen und anschließend mit Kaliumcarbonat getrocknet. Nach Abdestillieren des Ethers erhält man die Acetylverbindung als farbloses Öl in 95 %iger Ausbeute.

Die Reduktion der Acetylverbindung wird analog den Beispielen 28 bis 37 mit Lithiumaluminiumhydrid durchgeführt.

Beispiel 42

Auf einer Tablettenpresse werden in üblicher Weise Tabletten folgender Zusammensetzung gepreßt :

40 mg Substanz des Beispiels 37
120 mg Maisstärke
13,50 mg Gelatine
45 mg Milchzucker
22,5 mg Talk
2,25 mg Aerosil® (chemisch reine Kieselsäure in submikroskopisch feiner Verteilung)
6,75 mg Kartoffelstärke (als 6 %iger Kleister)

Beispiel 43

In üblicher Weise werden Dragees folgender Zusammensetzung hergestellt :

20 mg Substanz des Beispiels 37
60 mg Kernmasse
60 mg Verzuckerungsmasse

Die Kernmasse besteht aus 9 Teilen Maisstärke, 3 Teilen Milchzucker und 1 Teil Luviskol® VA 64 ("Vinylpyrrolidon-Vinylacetat-Mischpolymerisat 60 : 40, vlg. Pharm. Ind. *1962,* 586). Die Verzuckerungsmasse besteht aus 5 Teilen Rohrzucker, 2 Teilen Maisstärke, 2 Teilen Calciumcarbonat und 1 Teil Talk. Die so hergestellten Dragees werden anschließend mit einem magensaftresistenten Überzug versehen.

Beispiel 44

10 g Substanz des Beispiels 37 (freie Base) werden in 5 000 ml Wasser unter Zusatz von NaCl gelöst und mit 1 N HCl auf pH 6,0 eingestellt, so daß eine blutisotonische Lösung entsteht. Jeweils 5 ml der Lösung werden in Ampullen gefüllt und sterilisiert.

**Ansprüche**

1. Substituierte 1,5-Diaminopentane der Formel I

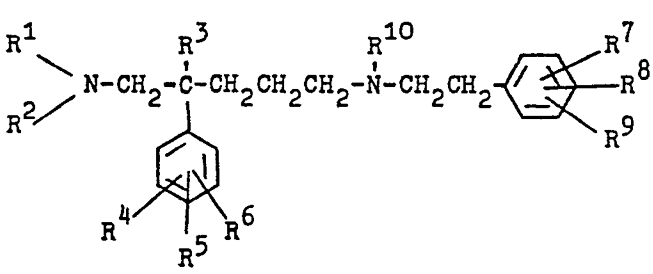

I,

worin

$R^1$ und $R^2$ Wasserstoffatome oder $C_{1-6}$-Alkylreste,

$R^3$ einen $C_{1-12}$-Alkylrest,

$R^4$-$R^9$ Wasserstoff- oder Halogenatome, $C_1$-$C_4$-Alkyl-, $C_1$-$C_4$-Alkoxy- oder Trifluormethylgruppen und

$R^{10}$ ein Wasserstoffatom oder eine Methylgruppe bedeuten,

sowie deren Salze mit physiologisch verträglichen Säuren.

2. Verfahren zur Herstellung substituierter 1,5-Diaminopentane der Formel I

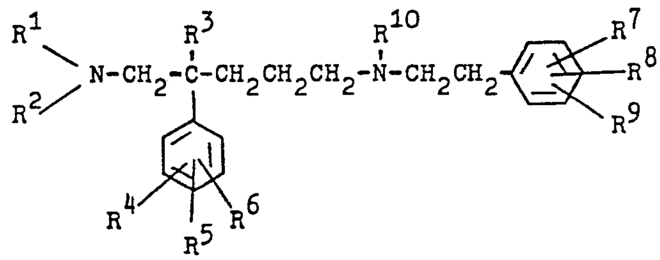

I,

worin

$R^1$ und $R^2$ Wasserstoffatome oder $C_{1-6}$-Alkylreste,

$R^3$ einen $C_{1-12}$-Alkylrest,

$R^4$-$R^9$ Wasserstoff- oder Halogenatome, $C_1$-$C_4$-Alkyl-, $C_1$-$C_4$-Alkoxy- oder Trifluormethylgruppen und

$R^{10}$ ein Wasserstoffatom oder eine Methylgruppe bedeuten,

sowie deren Salze mit physiologisch verträglichen Säuren, dadurch gekennzeichnet, daß man basisch substituierte Phenylacetonitrile der Formel II

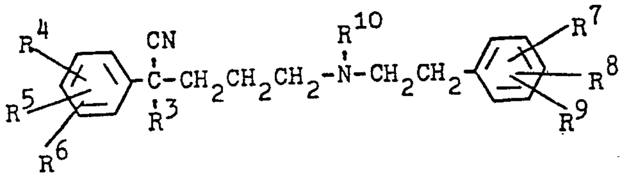

II,

worin $R^3$-$R^{10}$ die angegebene Bedeutung besitzen, reduziert und die so erhaltenen Verbindungen — falls gewünscht — mono- oder dialkyliert und gegebenenfalls anschließend in ihre Salze mit physiologisch verträglichen Säuren überführt.

3. Arzneimittel enthaltend eine Verbindung gemäß Anspruch 1.

4. Verwendung einer Substanz gemäß Anspruch 1 bei der Behandlung von Herzrhythmusstörungen.

5. Substanz gemäß Anspruch 1 zur Verwendung bei der Bekämpfung von Krankheiten.

**Claims**

1. A substituted 1,5-diaminopentane of the formula I

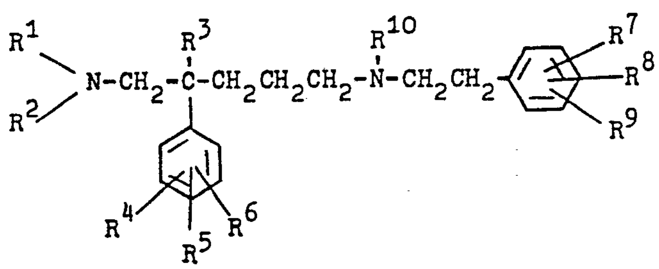

I,

where

$R^1$ and $R^2$ are each hydrogen or $C_1$-$C_6$-alkyl,

$R^3$ is $C_1$-$C_{12}$-alkyl,

$R^4$, $R^5$, $R^6$, $R^7$, $R^8$ and $R^9$ are each hydrogen, halogen, $C_1$-$C_4$-alkyl, $C_1$-$C_4$-alkoxy or trifluoromethyl and

$R^{10}$ is hydrogen or methyl, and its salts with physiologically tolerated acids.

2. A process for the preparation of a substituted 1,5-diaminopentane of the formula I

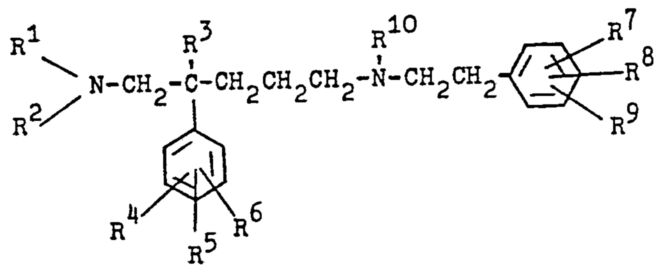

I,

where

$R^1$ and $R^2$ are each hydrogen or $C_1$-$C_6$-alkyl,

$R^3$ is $C_1$-$C_{12}$-alkyl,

$R^4$, $R^5$, $R^6$, $R^7$, $R^8$ and $R^9$ are each hydrogen, halogen, $C_1$-$C_4$-alkyl, $C_1$-$C_4$-alkoxy or trifluoromethyl and

$R^{10}$ is hydrogen or methyl, and its salts with physiologically tolerated acids, wherein a basically substituted phenylacetonitrile of the formula II

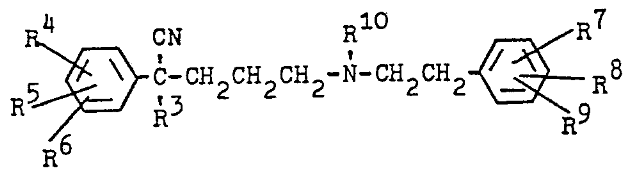

II,

where $R^3$, $R^4$, $R^5$, $R^6$, $R^7$, $R^8$, $R^9$ and $R^{10}$ have the above meanings, is reduced and the resulting compound, which may, if desired, be first monoalkylated or dialkylated, is, if appropriate, then converted into a salt with a physiologically tolerated acid.

3. A drug containing a compound of the formula I as claimed in claim 1.

4. The use of a substance as claimed in claim 1 in the treatment of cardiac arrhythmias.

5. A substance as claimed in claim 1 for use in the treatment of disorders.

**Revendications**

1. Diamino-1,5 pentanes substitués de la formule I

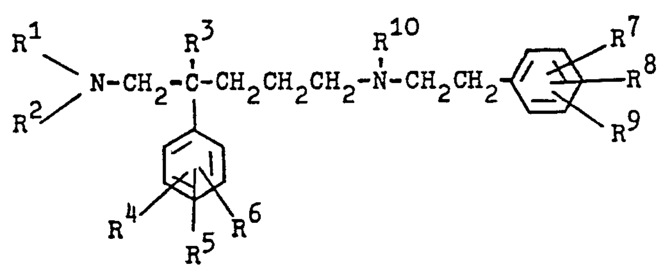

I,

dans laquelle

$R^1$ et $R^2$ désignent chacun un atome d'hydrogène ou un radical alcoyle en $C_1$ à $C_6$,

$R^3$ représente un radical alcoyle en $C_1$ à $C_{12}$,

$R^4$ à $R^9$ désignent chacun un atome d'hydrogène ou d'halogène, un radical alcoyle en $C_1$ à $C_4$ ou alcoxy en $C_1$ à $C_4$ ou un groupe trifluorométhyle et

$R^{10}$ représente un atome d'hydrogène ou un radical méthyle,

ainsi que leurs sels d'acides physiologiquement acceptables.

2. Procédé de préparation de diamino-1,5 pentanes substitués de la formule I

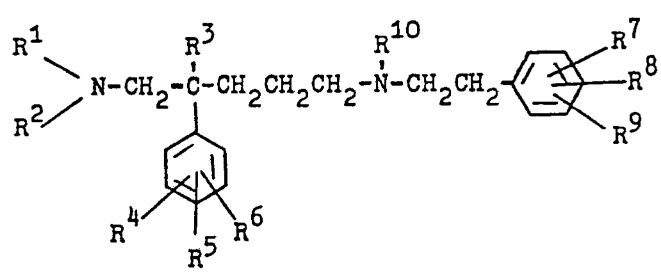

I,

dans laquelle

$R^1$ et $R^2$ désignent chacun un atome d'hydrogène ou un radical alcoyle en $C_1$ à $C_6$,

$R^3$ représente un radical alcoyle en $C_1$ à $C_{12}$,

$R^4$ à $R^9$ désignent chacun un atome d'hydrogène ou d'halogène, un radical alcoyle en $C_1$ à $C_4$ ou alcoxy en $C_1$ à $C_4$ ou un groupe trifluorométhyle et

$R^{10}$ représente un atome d'hydrogène ou un radical méthyle,

ainsi que de leurs sels d'acides physiologiquement acceptables, caractérisé en ce que l'on réduit des phényl-acéto-nitriles à substitution basique, de la formule II

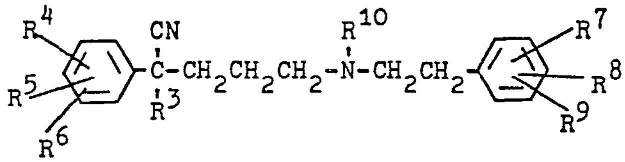

II,

dans laquelle les substituants $R^3$ à $R^{10}$ possèdent la signification définie, les composés formés pouvant, si on le désire, être soumis à une mono- ou une di-alcoylation, puis transformés éventuellement en sels d'acides physiologiquement acceptables.

3. Médicament, contenant un composé selon la revendication 1.

4. Utilisation d'un composé selon la revendication 1 pour le traitement de troubles du rythme cardiaque.

5. Composé selon la revendication 1, utilisé pour le traitement de maladies.